# EUROPEAN PATENT APPLICATION

(11) **EP 4 563 622 A1**
(43) Date of publication of application: **04.06.2025**
(21) Application number: 23846455.6
(22) Date of filing: 24.07.2023
(51) Int. Cl.: C08G 77/26, A61L 9/01, B01J 20/26, B01J 20/28, B01J 20/30, C08G 79/00, D06M 15/643

(54) **POLYSILOXANE, PRODUCTION METHOD FOR SAME, AND DEODORIZING AGENT CONTAINING SAME**

(30) Priority: 25.07.2022 JP 2022117668; 10.02.2023 JP 2023018847
(71) Applicant: Tosoh Corporation, Yamaguchi 746-8501 (JP); Sagami Chemical Research Institute, Ayase-shi Kanagawa 252-1193 (JP)
(72) Inventor: SUDO, Yukinori, Shunan-shi, Yamaguchi 746-8501 (JP); KOBAYASHI, Osamu, Ayase-shi, Kanagawa 252-1193 (JP); FUKAWA, Marina, Ayase-shi, Kanagawa 252-1193 (JP)
(74) Representative: Vossius & Partner Patentanwälte Rechtsanwälte mbB
(86) International application number: PCT/JP2023/027011
(87) International publication number: WO 2024/024725

(57) **Abstract**

The present invention provides a waterproof deodorant with excellent removal rate. An aminooxyalkyl-containing polysiloxane having a structural unit represented by the formula (1) is used.

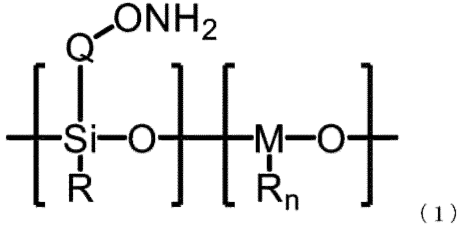

## Description

### TECHNICAL FIELD

The present invention relates to polysiloxane, a method for its production and a deodorant containing the same.

### BACKGROUND ART

Aldehydes such as acetaldehyde and formaldehyde are typical odorous substances in the living environment and can be a source of unpleasant odor even at low concentrations because of their very low detection thresholds. It is known that these aldehydes emitted from synthetic resins or plywood in buildings and automobiles or contained in cigarette smoke are responsible for sick building syndrome and sick car syndrome. Because these aldehydes are suspected to be carcinogenic, and everyday exposure to them is a risk to human health, the Ministry of Health, Labor and Welfare of Japan has set guideline values of concentration levels of acetaldehyde and formaldehyde in indoor air at 0.03 ppm and 0.08 ppm, respectively. Hence, there is a demand for means to remove aldehydes quickly and persistently.

Porous inorganic materials widely used as deodorants such as silica gel and activated carbon cannot remove lower aldehydes having low boiling points such as acetaldehyde and formaldehyde effectively. Therefore, aldehyde scavengers comprising hydrazine derivatives, amines, amino acids or urea derivatives are proposed to remove aldehydes by chemical reaction with aldehydes (Patent Documents 1 to 3).

### PRIOR ART DOCUMENTS

### PATENT DOCUMENTS

Patent Document 1: JP-A-H11-299878
Patent Document 2: JP-A-2000-300652
Patent Document 3: JP-A-2007-215818

### DISCLOSURE OF INVENTION

### TECHNICAL PROBLEM

The aldehyde scavengers disclosed in these documents cannot remove aldehydes efficiently enough and are not waterproof enough to withstand rinsing with water or washings without a performance loss due to dissolution into water when applied onto resin or textile substrates.

Considering the above-mentioned background art, the present invention aims to provide a polysiloxane which can remove aldehydes more effectively than conventional aldehyde scavengers even after rinsing with water or washings, a method for producing it, and a deodorant containing it.

### SOLUTION TO PROBLEM

As a result of their extensive research to solve the above-mentioned problem, the present inventors found the polysiloxane described below solves the above-mentioned problem and accomplished the present invention.

Namely, the present invention is embodied in the followings.

[1] An aminooxyalkyl-containing polysiloxane having a structural unit represented by the following formula (1): 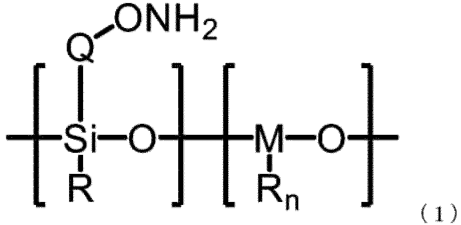 (wherein each R is independently a C₁₋₄ alkyl group, a C₁₋₄ alkoxy group or a hydroxy group, each Q is independently a group selected from the group consisting of -CH₂-, - CH₂CH₂- and -CH₂-Ra-CH₂- (wherein Ra is a C₁₋₁₈ alkylene group in which two or more interspaced -CH₂- groups may each be independently replaced by -O-, -(C=O)O-, - O(C=O)-, -O(C=O)-O-, -C(=O)-NH-, -NH-(C=O)-, -CH=CH- or -C=C-, and in which a hydrogen atom in the alkylene group may be substituted with a C₁₋₃ alkyl group, M is an aluminum atom, a silicon atom or a titanium atom, and n is an integer of 1 or 2, provided that when M is an aluminum atom, n is 1, and when M is a silicon atom or a titanium atom, n is 2).
[2] The aminooxyalkyl-containing polysiloxane according to [1], wherein each R is independently a methyl group, a methoxy group, an ethoxy group, a propyloxy group, an isopropyloxy group or a hydroxy group.
[3] The aminooxyalkyl-containing polysiloxane according to [1] or [2], wherein M is a silicon atom, and n is 2.
[4] The aminooxyalkyl-containing polysiloxane according to any one of [1] to [3], which has a volume median diameter of at most 300 nm.
[5] A method for producing the aminooxyalkyl-containing polysiloxane as defined in [1], which comprises copolymerizing a compound represented by the following formula (2) with a compound represented by the following formula (4) and/or a compound represented by the following formula (5) in the presence of water: 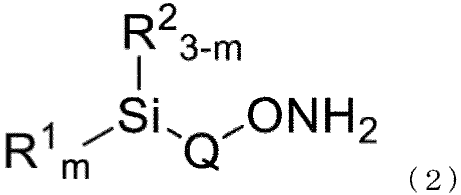 (wherein each R¹ is independently a C₁₋₄ alkyl group, each R² is independently a C₁₋₄ alkoxy group, Q is the same as defined above, and m is an integer of from 0 to 2) 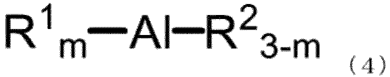 (wherein R¹, R² and m are the same as defined above) 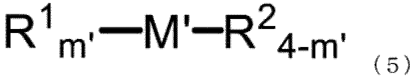 (wherein R¹ and R² are the same as defined above, M' is a silicon atom or a titanium atom, and m' is an integer of from 0 to 3).
[6] A method for producing the aminooxyalkyl-containing polysiloxane as defined in [1], which comprises copolymerizing a compound represented by the following formula (2) with a compound represented by the following formula (3) in the presence of water: 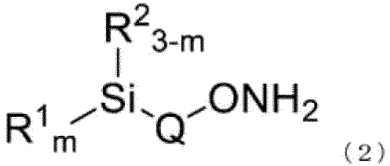 (wherein each R¹ is independently a C₁₋₄ alkyl group, each R² is independently a C₁₋₄ alkoxy group, Q is the same as defined above, and m is an integer of from 0 to 2) 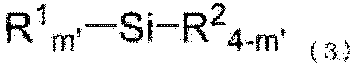 (wherein R¹ and R² are the same as defined above, and m' is an integer of from 0 to 3).
[7] The method for producing the aminooxyalkyl-containing polysiloxane according to [5], wherein the ratio (molar ratio) of the total molar amount of the compound represented by the formula (4) and the compound represented by the formula (5) to the molar amount of the compound represented by the formula (2) is such that the total molar amount of the compound represented by the formula (4) and the compound represented by the formula (5) is from 0.001 to 1,000 moles per 1 mole of the compound represented by the formula (2).
[8] The method for producing the aminooxyalkyl-containing polysiloxane according to [6], wherein the ratio (molar ratio) of the molar amount of the compound represented by the formula (3) to the molar amount of the compound represented by the formula (2) is such that the molar amount of the compound represented by the formula (3) is from 0.001 to 1,000 moles per 1 mole of the compound represented by the formula (2).
[9] A deodorant comprising the aminooxyalkyl-containing polysiloxane as defined in [1] or [2].
[10] A deodorant article comprising the aminooxyalkyl-containing polysiloxane as defined in [1] or [2] and a substrate carrying the aminooxyalkyl-containing polysiloxane.
[11] The deodorant article according to [10], wherein the substrate is in the form of fiber, sheet, beads, sponge or board.
[12] A method for removing an aldehyde, which comprises bringing the deodorant article as defined in [10] into contact with air containing an aldehyde.

### ADVANTAGEOUS EFFECTS OF INVENTION

A deodorant containing the aminooxyalkyl-containing polysiloxane of the present invention can remove aldehydes quickly and persistently, and as a result, can improve the living environment by decreasing aldehydes harmful to humans.

### DESCRIPTION OF EMBODIMENTS

Now, the present invention will be described in detail. Herein, "to" expresses a numerical range including the figures before and after "to" as the lower limit and the upper limit.

The aminooxyalkyl-containing polysiloxane of the present invention is characterized by having a structural unit represented by the above-mentioned formula (1).

In the formula (1), each R is independently a C₁₋₄ alkyl group, a C₁₋₄ alkoxy group or a hydroxy group.

The C₁₋₄ alkyl group is not particularly restricted and may, for example, be a methyl group, an ethyl group, a propyl group, an isopropyl group, a butyl group, a 2-methylpropyl group, a 1-methylpropyl group or a tert-butyl group.

The C₁₋₄ alkoxy group is not particularly restricted and may, for example, be a methoxy group, an ethoxy group, a propyloxy group, an isopropyloxy group, a butoxy group, a 2-methylpropyloxy group, a 1-methylpropyloxy group or a tert-butoxy group.

Among them, R is preferably a methyl group, a methoxy group, an ethoxy group, a propyloxy group, an isopropyloxy group or a hydroxy group.

Each Q is independently a group selected from the group consisting of -CH₂-, - CH₂CH₂- and -CH₂-Ra-CH₂- (wherein Ra is a C₁₋₁₈ alkylene group in which two or more interspaced -CH₂- groups may each be independently replaced by -O-, -(C=O)O-, - O(C=O)-, -O(C=O)-O-, -C(=O)-NH-, -NH-(C=O)-, -CH=CH- or -C≡C-, and in which a hydrogen atom in the alkylene group may be substituted with a C₁₋₃ alkyl group).

Q is not particularly restricted, and specific examples include structures represented by the following chemical formulae (Q-1) to (Q-95). 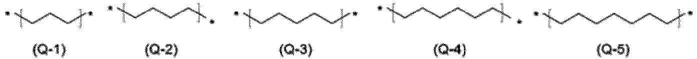 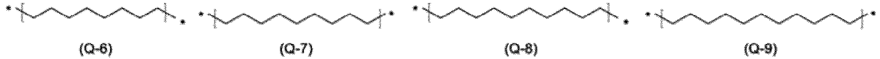 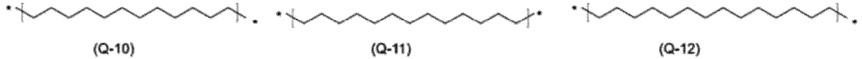 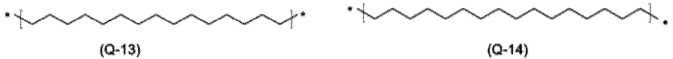 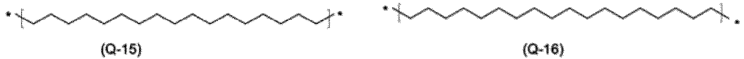 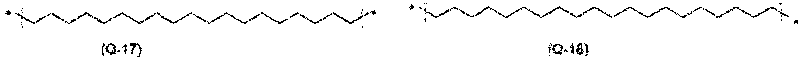 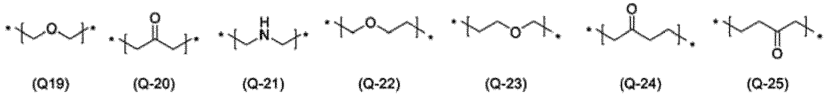 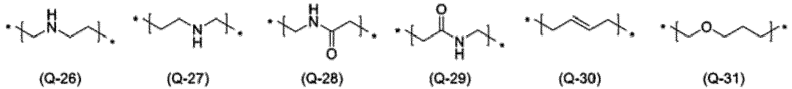 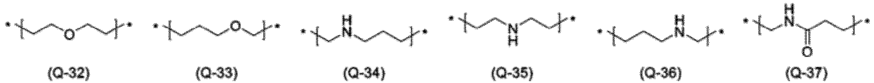 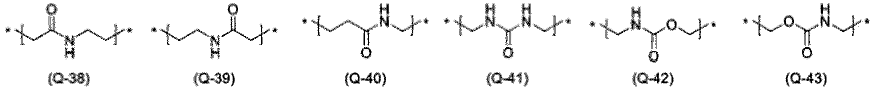 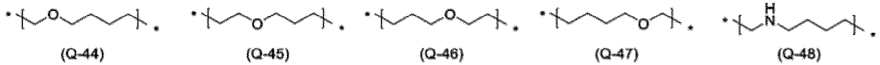 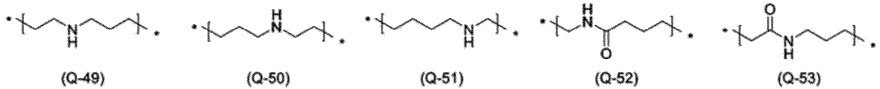 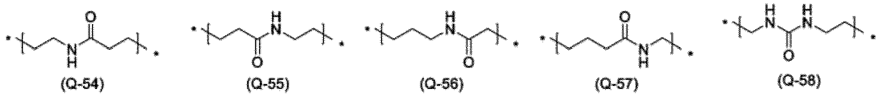 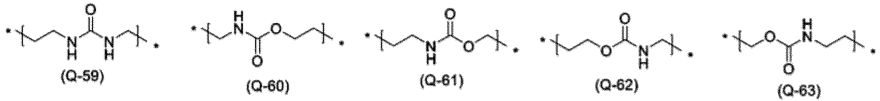 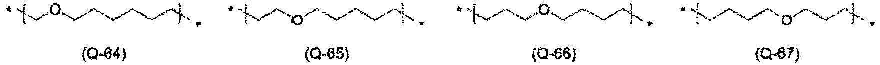 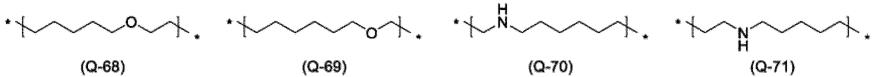 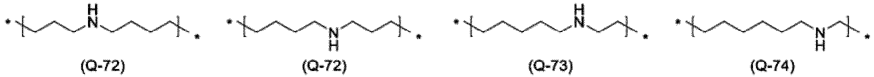 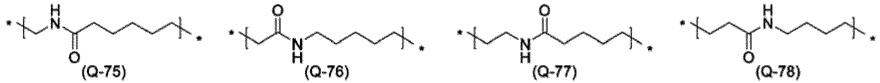 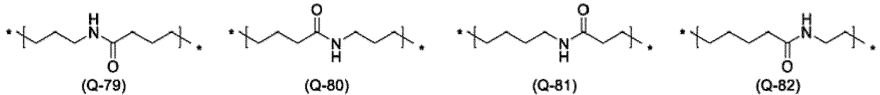 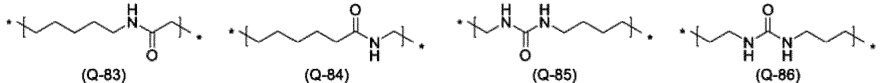 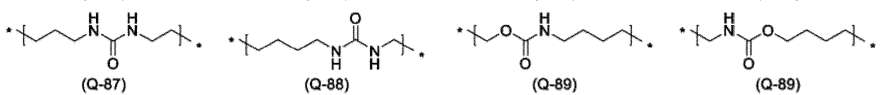 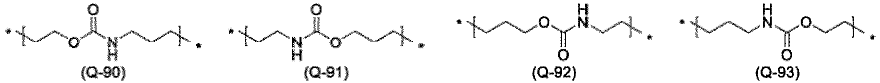 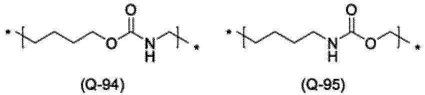 [in the chemical formulae (Q-1) to (Q-95), a hydrogen atom in an alkylene group may be substituted with a C₁₋₃ alkyl group, and * represents a bond shown in the formula (1).]

The C₁₋₃ alkyl group may, for example, be a methyl group, an ethyl group, a propyl group or an isopropyl group.

In the formula (1), M is an aluminum atom, a silicon atom or a titanium atom. n is an integer of 1 or 2. When M is an aluminum atom, n is 1, and when M is a silicon atom or a titanium atom, n is 2. When n is 1, M is bonded to one R. When n is 2, M is bonded to two Rs.

In the above-mentioned formula (1), it is preferred that M is a silicon atom, and n is 2. Namely, an aminooxyalkyl-containing polysiloxane represented by the following formula (1a) is preferred. 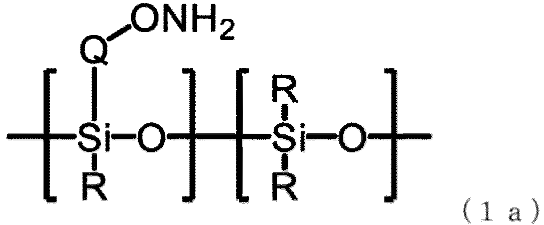 (wherein R and Q are the same as R and Q in the formula (1).)

The aminooxyalkyl-containing polysiloxane represented by the above-mentioned formula (1) is preferably an aminooxyalkyl-containing polysiloxane represented by the following formula (1'). 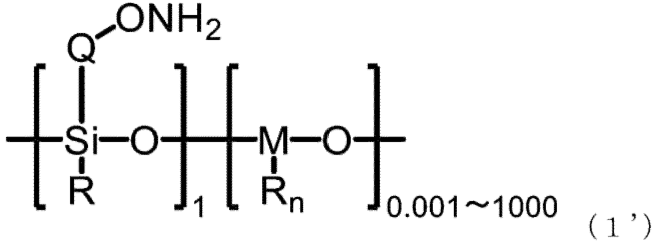 (wherein R, Q, M and n are the same as R, Q, M and n in the formula (1).)

The aminooxyalkyl-containing polysiloxane represented by the above-mentioned formula (1a) is preferably an aminooxyalkyl-containing polysiloxane represented by the following formula (1a'). 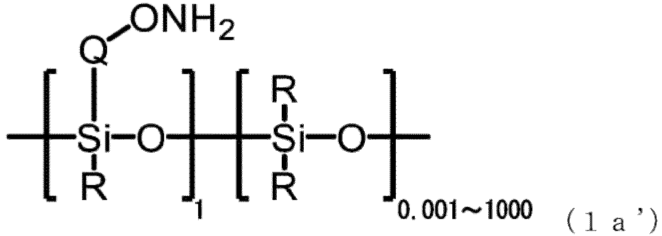 (wherein R and Qn are the same as R and Q in the formula (1).)

The aminooxyalkyl-containing polysiloxane of the present invention is a copolymer of a monomer having an aminooxyalkyl group and a monomer having no aminooxyalkyl group. The above-mentioned formula (1') or (1a') means that the molar ratio of a monomer having an aminooxyalkyl group : a monomer having no aminooxyalkyl group is 1 : from 0.001 to 1,000.

The ratio (molar ratio) of a monomer having an aminooxyalkyl group : a monomer having no aminooxyalkyl group is such that the ratio of "the molar amount of the monomer having an aminooxyalkyl group" : "the molar amount of the monomer having no aminooxyalkyl group" is preferably 1 : from 0.001 to 1,000, more preferably 1 : from 0.01 to 100, more preferably 1 : from 0.01 to 10, more preferably 1 : from 0.01 to 4, more preferably 1 : from 0.01 to 2, in view of the ability to capture aldehydes and easy production.

The volume median diameter of the aminooxyalkyl-containing polysiloxane of the present invention is preferably at most 1,000 nm, more preferably at most 500 nm, more preferably at most 300 nm, further preferably at most 200 nm, in view of workability. The above-mentioned average particle diameter is a volume average particle diameter measured by dynamic light scattering.

The volume median diameter is preferably 1 to 1,000 nm, more preferably 5 to 500 nm, more preferably 10 to 300 nm, further preferably 10 to 200 nm in view of the ability to capture aldehydes.

The aminooxyalkyl-containing polysiloxane of the present invention may be produced by any methods without particular restriction, for example, by copolymerizing a compound represented by the formula (2) with a compound represented by the formula (4) and/or a compound represented by the formula (5) or copolymerizing a compound represented by the formula (2) with a compound represented by the formula (3), in the presence of water.

The compounds represented by the formulae (2) to (5) may each be a mixture containing two or more species of compounds in a certain ratio.

In the compounds represented by the formulae (2) to (5), R¹ is a C₁₋₄ alkyl group, and R² is a C₁₋₄ alkoxy group.

The C₁₋₄ alkyl group is not particularly restricted and may, for example, be a methyl group, an ethyl group, a propyl group, an isopropyl group, a butyl group, a 2-methylpropyl group, a 1-methylpropyl group or a tert-butyl group.

The C₁₋₄ alkoxy group is not particularly restricted and may, for example, be a methoxy group, an ethoxy group, a propyloxy group, an isopropyloxy group, a butoxy group, a 2-methylpropyloxy group, a 1-methylpropyloxy group or a tert-butoxy group.

In the compounds represented by the formulae (2) and (4), m is an integer of from 0 to 2.

In the compound represented by the formula (5), M' is a silicon atom or a titanium atom, and m' is an integer of from 0 to 3.

In the compound represented by the formula (3), m' is an integer of from 0 to 3.

Among them, for efficient copolymerization, R¹ is preferably a methyl group, R² is preferably a methoxy group, an ethoxy group, a propyloxy group or an isopropyloxy group, m is preferably 0 or 1, and m' is preferably 0, 1 or 2.

The copolymerization is carried out in the presence of water, and hydrolysis of the metal alkoxide moieties of the compounds represented by the formulae (2) to (5) into metal hydroxides firstly occur and then followed by dehydration condensation.

The amount of water is not particularly restricted, but is preferably at least 1 molar equivalent, more preferably from 1 to 10 molar equivalents, more preferably from 1 to 5 molar equivalents, in relation to a compound represented by the formula (2), for efficient copolymerization.

In the copolymerization, water may be used as a reaction accelerator and a solvent, and, if necessary, a solvent other than water may be added.

The solvent other than water is not particularly restricted, but is preferably a solvent miscible with water, such as methanol, ethanol, propanol (alcohol solvents), tetrahydrofuran, dioxane (ether solvents), acetonitrile, dimethylformamide, dimethyl sulfoxide or hexamethylphosphoramide.

The solvent other than water may be a single solvent or a mixture of solvents.

The amount of the solvent other than water is not particularly restricted and is usually from 1 to 100 parts by weight per 1 part by weight of the compound represented by the formula (2).

The molar ratio of the compound represented by the formula (2) to the compound represented by the formula (4) and/or the compound represented by the formula (5) in the copolymerization may be adjusted arbitrarily in accordance with the intended use without any particular restrictions and is such that the total molar amount of the compound represented by the formula (4) and/or the compound represented by the formula (5) is preferably from 0.001 to 1,000, more preferably from 0.01 to 100, more preferably from 0.01 to 10, more preferably from 0.01 to 4 , more preferably from 0.01 to 2, per 1 mole of the compound represented by the formula (2).

The molar ratio of the compound represented by the formula (2) to the compound represented by the formula (3) in the copolymerization may be adjusted arbitrarily in accordance with the intended use without any particular restrictions and is such that the moles of the compound represented by the formula (3) per 1 mole of the compound represented by the formula (2) is preferably from 0.001 to 1,000, more preferably from 0.01 to 100, more preferably from 0.01 to 10, more preferable from 0.01 to 4, more preferably from 0.01 to 2.

The copolymerization is usually carried out at a temperature of from -20 to 200°C, preferably from 0 to 150°C, in view of the production equipment.

The reaction time of the copolymerization varies depending on the concentrations of the compounds represented by the formulae (3) to (5), the amount of water and the reaction temperature and is usually several minutes to 14 days.

The copolymerization may be any type of copolymerization, such as random copolymerization, alternating copolymerization, block copolymerization or graft copolymerization.

The molecule of the aminooxy-containing polysiloxane of the present invention may be in any shape without particular restrictions and may be linear, branched or cyclic.

After the copolymerization, a mixture containing the aminooxyalkyl-containing polysiloxane of the present invention is obtained. The mixture may be used directly, or, if necessary, after purification by distillation, reprecipitation, liquid-liquid separation, filtration or column chromatography or the like.

The deodorant of the present invention is characterized in that it comprises the aminooxyalkyl-containing polysiloxane of the present invention.

The deodorant of the present invention may comprise a solvent, an acid, a base, a binder, a thickener, an anti-foaming agent, a surfactant, an anti-fungus agent, an antiseptic agent or the like, in addition to the aminooxyalkyl-containing polysiloxane.

The solvent is not particularly restricted and may, for example, be water, methanol, ethanol, propanol or acetonitrile. In view of safety to humans, water or ethanol is preferred (hereinafter a mixture of the deodorant and a solvent is referred to a deodorant liquid).

The concentration of the aminooxyalkyl-containing polysiloxane in the deodorant of the present invention is not particularly restricted, but is preferably from 0.1 to 50 wt%, more preferably from 1 to 30 wt%.

The content of an acid or a base, if any, in the deodorant of the present invention is not particularly restricted and is preferably from 0.001 to 10 molar equivalents, more preferably from 0.005 to 5 molar equivalents, more preferably from 0.01 to 2 molar equivalents, per 1 mole of the -ONH₂ group.

The concentration of a binder, a thickener, an anti-foaming agent, a surfactant, an anti-fungus agent or an antiseptic agent, if any, in the a deodorant of the present invention is not particularly restricted and is preferably from 0.1 to 30 wt%, more preferably from 0.2 to 25 wt%, more preferably from 0.2 to 10 wt%, in relation of the entire deodorant liquid, respectively.

The deodorant of the present invention is obtained by any method without particular restrictions and may, for example, be obtained by mixing the aminooxyalkyl-containing polysiloxane of the present invention and a solvent, by stirring.

The deodorant of the present invention may be used in any ways with no particular restrictions, for example, by applying or spraying the deodorant onto a substrate or by spraying the deodorant onto the order source.

The deodorant article of the present invention comprises the aminooxyalkyl-containing polysiloxane and a substrate carrying the aminooxyalkyl-containing polysiloxane.

As the substrate in the deodorant article of the present invention, fiber, sheet, beads, sponge or a board may, for example, be mentioned, though there are no particular restrictions.

More specific examples are polyester fiber, polyamide fiber, polyacrylonitrile fiber, polypropylene fiber, polyethylene fiber, polyvinyl chloride fiber, fluorine fiber, aramid fiber, sulfone fiber, rayon, acetate, cotton, wool, silk, hemp, glass fiber, carbon fiber, ceramic fiber, a fiber blend of these fibers, a woven fabric of these fibers, a knitted fabric of these fibers, a rope of these fibers, a string of these fibers, or a sheet, beads, sponge of a polyester resin, a polyamide resin, a polyethylene resin, a polypropylene resin, a fluororesin, a silicone resin, a polyimide resin, a polyvinyl chloride resin, natural rubber or a synthetic rubber, wood, plywood and a plaster board.

The deodorant article of the present invention may be used for any purposes and may be used for clothing, curtains, carpets, wall coverings, automobile interior materials or furniture.

The deodorant article of the present invention may be produced by any methods without particular restrictions, for example, by loading the aminooxyalkyl-containing polysiloxane of the present invention or the deodorant onto a substrate.

The aminooxyalkyl-containing polysiloxane or the deodorant may be loaded onto a substrate by any method without particular restrictions, for example, by applying the deodorant liquid onto the substrate or by immersing the substrate in the deodorant liquid.

The amount of the aminooxyalkyl-containing polysiloxane on the substrate can be adjusted arbitrarily in accordance with the intended use without any particular restrictions, but is preferably from 0.1 to 100 g/m², more preferably from 0.5 to 50 g/m².

After loading of the aminooxyalkyl-containing polysiloxane or the deodorant onto the substrate, the resulting deodorant article may be dried, if necessary. The drying conditions can be adjusted arbitrarily in accordance with the intended use without any particular restrictions. For example, the temperature is from 0 to 200°C, and the time is from several minutes to 48 hours.

### BRIEF DESCRIPTION OF DRAWINGS

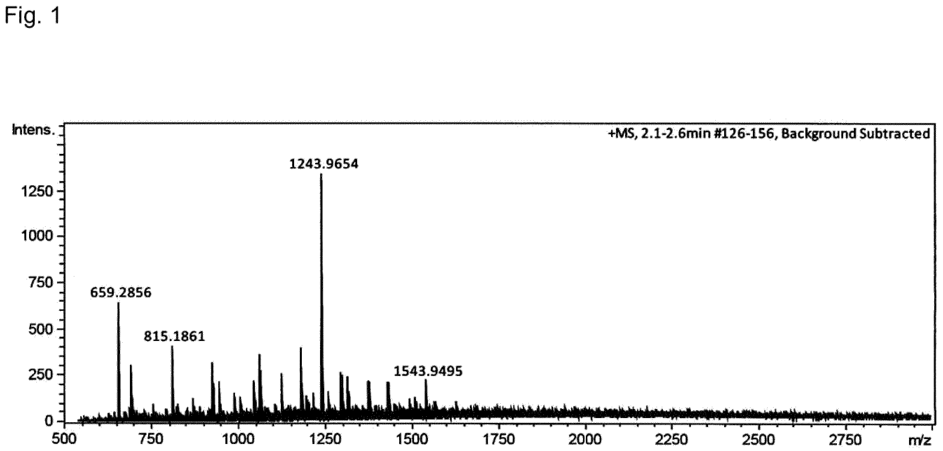

[Fig. 1] The mass spectrum of the aminooxyalkyl-containing polysiloxane obtained in Example 1.

### EXAMPLES

Now, the present invention will be described in further detail with reference to Examples. However, it should be understood that these Examples are intended to help understand the present invention, and the present invention is by no means restricted thereto. As the reagents, commercially available products were used unless otherwise noted.

For the compounds used in the Examples, the following abbreviations are used.

O-[3-(Triethoxysilyl)propyllhydroxylamine = Si-1
O-[3-(Trimethoxysilyl)propyl]hydroxylamine = Si-2
O-[3-[Diethoxy(methyl)silyl]propyl]hydroxylamine = Si-3
O-[3-[Dimethoxy(methyl)silyl]propyl]hydroxylamine = Si-4
1-[2-(Aminooxy)ethyl]-3-[3-(trimethoxysilyl)propyl]urea = Si-5
1-[2-(Aminooxy)ethyl]-N-[3-(trimethoxysilyl)propyl] carbamate = Si-6
2-Aminooxy-N-[3-(trimethoxysilyl)propyl]acetamide = Si-7
2-Aminooxy-N-[3-(trimethoxysilyl)propyl]propenamide = Si-8
Tetraethoxysilane = TEOS
Methyltriethoxysilane = MTEOS

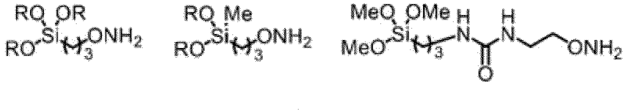

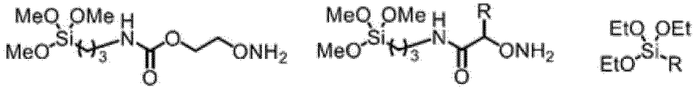

### EXAMPLE 1

### <Preparation of Deodorant>

In a graduated centrifuge tube, Si-1 (1.62 g, 8.28 mmol), TEOS (0.513 g, 2.46 mmol) and water (21.2 g) were stirred at room temperature for 7 days to obtain a translucent dispersion containing an aminooxyalkyl-containing polysiloxane of the present invention. The progress of copolymerization was confirmed by the change of the reaction system from two phases to a homogeneous single phase. The molecular weight of the resulting aminooxyalkyl-containing polysiloxane was analyzed with a mass spectrometer (microTOF, manufactured by Bruker Daltonics), and the spectrum shown in Fig. 1 was obtained, which indicates production of a polymer. The volume median diameter of the aminooxyalkyl-containing polysiloxane was measured by dynamic light scattering with an instrument (model: ELSZ-2000) manufactured by OTSUKA ELECTRONICS CO., LTD., and found to be 5 nm.

### <Preparation of Deodorant Article>

0.1 g of the translucent dispersion obtained by the above-mentioned reaction was applied onto a 5 cm × 10 cm 100% cotton cloth and dried in a hot-air dryer (DRJ433DA, manufactured by the ADVANTECH group) at 60°C for 1 hour to obtain a deodorant article carrying the aminooxyalkyl-containing polysiloxane (the amount of the aminooxy group = 35 µmol).

### <Acetaldehyde Removal Test>

The deodorant article was sealed in a 10 L Tedlar bag, and 5 L of nitrogen gas containing about 10 ppm acetaldehyde was injected. After 2 hours of standing at room temperature, the gas in the Tedlar bag was passed through a 2,4-dinitrophenylhydrazine (DNPH) cartridge column (Precep-C DNPH, manufactured by FUJIFILM Wako Pure Chemical Corporation) to adsorb the aldehyde in the gas on it. The DNPH-aldehyde condensate was eluted from the column with acetonitrile. The eluate was analyzed with a liquid chromatograph (LC-2030C Plus, manufactured by Shimadzu Corporation) to determine the concentration of the acetaldehyde. The aldehyde removal rate [%] was calculated from the following formula. Acetaldehyde removal rate [%] = [(Initial acetaldehyde concentration - Remaining acetaldehyde concentration) / Initial acetaldehyde concentration] × 100

### EXAMPLES 2 TO 35 AND REFERENCE EXAMPLES 1 TO 6

Examples 2 to 35 and Reference Examples 1 to 6 were carried out in the same manner as Example 1 except that deodorants were prepared by changing kinds and amounts of the starting materials and production conditions as shown in Table 1. Deodorant articles were prepared so that 35 µmol of aminooxy group was carried per cloth, as in Example 1. In Table 1, compounds represented by the formula (2) are categorized as monomer A, and compounds represented by the formulae (3) to (5) are categorized as monomer B. The results of determination of volume median diameter and aldehyde removal rate are shown in Table 2.

### COMPARATIVE EXAMPLE 1

A deodorant article was prepared in the same manner as in Example 1 except that 0.1 g of 5% aqueous adipic dihydrazide was applied onto a 5 cm × 10 cm 100% cotton cloth, instead of 0.1 g of the translucent dispersion used in Example 1, and the aldehyde removal rate was measured.

### COMPARATIVE EXAMPLE 2

A deodorant article was prepared in the same manner as in Example 1 except that 0.2 g of 5% aqueous adipic dihydrazide was applied onto a 5 cm × 10 cm 100% cotton cloth, instead of 0.1 g of the translucent dispersion used in Example 1, and the aldehyde removal rate was measured.

The results of Examples 1 to 35, Reference Examples 1 to 6 and Comparative Examples 1 to 2 are shown in Table 2. It is clear from Table 2 that the deodorants of the present invention showed higher aldehyde removal rates than conventional deodorants.

**[Table 1]**

| | Starting materials (wt%) | | | | | Production conditions |
|---|---|---|---|---|---|---|
| | Monomer A | Monomer B | Water | Ethanol | Additive | |
| Ex. 1 | Si-1 (8.3) | TEOS (2.2) | 89.5 | - | - | Room temperature, 7 days |
| Ex. 2 | Si-1 (8.3) | TEOS (2.2) | 89.5 | - | - | 50°C, 2 days |
| Ex. 3 | Si-1 (8.3) | TEOS (2.2) | 85.3 | - | 5% Hydrochloric acid (4.2) | Room temperature, 2 days |
| Ex. 4 | Si-1 (8.3) | TEOS (2.2) | 88.5 | - | Boric acid (1.1) | Room temperature, 5 days |
| Ex. 5 | Si-1 (8.3) | TEOS (2.2) | - | - | 0.27% Aqueous sodium hydroxide (89.5) | Room temperature, 3 hours |
| Ex. 6 | Si-1 (8.3) | TEOS (2.2) | 50.0 | 39.5 | - | Room temperature, 2 days |
| Ex. 7 | Si-1 (8.3) | TEOS (2.2) | 47.7 | 37.6 | 5% Hydrochloric acid (4.2) | Room temperature, 24 hours |
| Ex. 8 | Si-1 (8.3) | TEOS (2.2) | 50.0 | 39.5 | - | 50°C, 10 hours |
| Ex. 9 | Si-1 (8.3) | TEOS (0.8) | 50.8 | 40.1 | - | Room temperature, 20 hours |
| Ex. 10 | Si-1 (8.3) | TEOS (1.1) | 50.6 | 40.0 | - | Room temperature, 20 hours |
| Ex. 11 | Si-1 (8.3) | TEOS (1.5) | 50.4 | 39.8 | - | Room temperature, 20 hours |
| Ex. 12 | Si-1 (8.3) | TEOS (1.8) | 50.2 | 39.6 | - | Room temperature, 20 hours |
| Ex. 13 | Si-1 (8.3) | TEOS (2.4) | 49.9 | 39.4 | - | Room temperature, 20 hours |
| Ex. 14 | Si-1 (8.3) | TEOS (2.9) | 49.6 | 39.2 | - | Room temperature, 20 hours |
| Ex. 15 | Si-1 (8.3) | TEOS (3.9) | 49.0 | 38.8 | - | Room temperature, 20 hours |
| Ex. 16 | Si-1 (8.3) | TEOS (4.4) | 48.8 | 38.5 | - | Room temperature, 20 hours |
| Ex. 17 | Si-1 (8.3) | TEOS (6.2) | 47.8 | 37.7 | - | Room temperature, 20 hours |
| Ex. 18 | Si-1 (8.3) | TEOS (7.3) | 47.2 | 37.2 | - | Room temperature, 20 hours |
| Ex. 19 | Si-1 (8.3) | MTEOS (1.9) | 50.2 | 39.6 | - | Room temperature, 20 hours |
| Ex. 20 | Si-1 (8.3) | Ti(OiPr)₄ (2.8) | 49.7 | 39.2 | - | Room temperature, 20 hours |
| Ex. 21 | Si-2 (8.8) | TEOS (0.3) | 90.9 | - | - | Room temperature, 24 hours |
| Ex. 22 | Si-2 (7.7) | TEOS (1.2) | 91.1 | - | - | Room temperature, 24 hours |
| Ex. 23 | Si-2 (6.8) | TEOS (2.2) | 91.0 | - | - | Room temperature, 18 hours |

**[Table 1] (continued)**

| | Starting materials (wt%) | | | | | Production conditions |
|---|---|---|---|---|---|---|
| | Monomer A | Monomer B | Water | Ethanol | Additive | |
| Ex. 24 | Si-2 (6.4) | TEOS (2.6) | 91.0 | - | - | Room temperature, 24 hours |
| Ex. 25 | Si-2 (5.6) | TEOS (1.8) | 92.6 | - | - | Room temperature, 22 hours |
| Ex. 26 | Si-2 (8.6) | TEOS (2.7) | 88.6 | - | 28% Aqueous ammonia (0.1) | Room temperature, 3 hours |
| Ex. 27 | Si-2 (7.3) | MTEOS (2.0) | 90.7 | - | - | Room temperature, 5 days |
| Ex. 28 | Si-2 (5.5) | MTEOS (1.5) | 93.0 | - | - | Room temperature, 15 hours |
| Ex. 29 | Si-2 (6.8) | Al(OiPr)₃ (2.9) | 90.2 | - | - | Room temperature, 20 hours |
| Ex. 30 | Si-3 (7.3) | TEOS (2.2) | 50.6 | 39.9 | - | Room temperature, 20 hours |
| Ex. 31 | Si-3 (7.3) | MTEOS (1.9) | 50.8 | 40.0 | - | Room temperature, 20 hours |
| Ex. 32 | Si-4 (6.3) | TEOS (2.2) | 51.2 | 40.3 | - | Room temperature, 20 hours |
| Ex. 33 | Si-4 (6.3) | MTEOS (1.9) | 51.3 | 40.5 | - | Room temperature, 20 hours |
| Ex. 34 | Si-5 (9.8) | TEOS (2.2) | 49.2 | 38.8 | - | Room temperature, 20 hours |
| Ex. 35 | Si-6 (9.9) | TEOS (2.2) | 49.1 | 38.8 | - | Room temperature, 20 hours |
| Ref. Ex. 1 | Si-1 (8.3) | - | 51.3 | 40.4 | - | Room temperature, 20 hours |
| Ref. Ex. 2 | Si-1 (8.3) | Al(OiPr)₃ (2.9) | 49.6 | 39.2 | - | Room temperature, 20 hours |
| Ref. Ex. 3 | Si-2 (8.6) | TEOS (2.7) | 88.7 | - | - | Room temperature, 9 days |
| Ref. Ex. 4 | Si-2 (6.8) | Ti(OiPr)₄ (2.8) | 50.5 | 39.9 | - | Room temperature, 20 hours |
| Ref. Ex. 5 | Si-7 (8.8) | TEOS (2.1) | 49.7 | 39.4 | - | Room temperature, 20 hours |
| Ref. Ex. 6 | Si-8 (9.3) | TEOS (2.2) | 49.4 | 39.1 | - | Room temperature, 20 hours |
| Com. Ex. 1 | 5% Aqueous adipic dihydrazide | | | | | - |
| Com. Ex. 2 | 5% Aqueous adipic dihydrazide | | | | | - |

**[Table 2]**

| | Molar ratio monomer B / monomer A | Volume median diameter [nm] | Acetaldehyde removal rate [%] |
|---|---|---|---|
| Ex. 1 | 0.29 | 5 | 99.8 |
| Ex. 2 | 0.29 | 3 | 93.3 |
| Ex. 3 | 0.29 | 4 | 97.8 |
| Ex. 4 | 0.29 | 4 | 99.9 |
| Ex. 5 | 0.29 | 5 | 74.5 |
| Ex. 6 | 0.29 | 4 | 99.6 |
| Ex. 7 | 0.29 | 10 | 83.7 |
| Ex. 8 | 0.29 | 6 | 99.7 |
| Ex. 9 | 0.10 | 3 | 81.6 |
| Ex. 10 | 0.15 | 3 | 82.8 |
| Ex. 11 | 0.20 | 8 | 93.3 |
| Ex. 12 | 0.26 | 7 | 98.0 |
| Ex. 13 | 0.33 | 5 | 99.5 |
| Ex. 14 | 0.40 | 7 | 99.2 |
| Ex. 15 | 0.50 | 3 | 99.0 |
| Ex. 16 | 0.59 | 5 | 98.5 |
| Ex. 17 | 0.80 | 9 | 98.8 |
| Ex. 18 | 1.00 | 10 | 99.7 |
| Ex. 19 | 0.29 | 7 | 91.1 |
| Ex. 20 | 0.29 | 255 | 91.3 |
| Ex. 21 | 0.03 | 4 | 99.4 |
| Ex. 22 | 0.15 | 4 | 99.5 |
| Ex. 23 | 0.29 | 22 | 99.6 |
| Ex. 24 | 0.37 | 1 | 99.4 |
| Ex. 25 | 0.29 | 17 | 97.5 |
| Ex. 26 | 0.29 | 16 | 99.4 |
| Ex. 27 | 0.29 | 17 | 97.5 |
| Ex. 28 | 0.22 | 84 | 99.3 |
| Ex. 29 | 0.29 | 67 | 98.7 |
| Ex. 30 | 0.29 | 8 | 92.0 |
| Ex. 31 | 0.29 | 7 | 72.8 |
| Ex. 32 | 0.29 | 4 | 91.3 |
| Ex. 33 | 0.29 | 4 | 80.5 |
| Ex. 34 | 0.29 | 10 | 72.9 |
| Ex. 35 | 0.29 | 8 | 77.2 |
| Ref. Ex. 1 | - | 5 | 65.5 |
| Ref. Ex. 2 | 0.29 | 506 | 97.8 |
| Ref. Ex. 3 | 0.29 | 1878 | 99.5 |
| Ref. Ex. 4 | 0.29 | 1959 | 95.7 |
| Ref. Ex. 5 | 0.29 | 6 | 67.9 |
| Ref. Ex. 6 | 0.29 | 11 | 63.2 |
| Com. Ex. 1 | - | - | 40.0 |
| Com. Ex. 2 | - | - | 55.5 |

### EXAMPLE 36

The deodorant article obtained in Example 1 was washed once or 5 times in accordance with the washing durability test (JIS L0217 103).

The deodorant article was assessed by the acetaldehyde removal test, as in Example 1, after one washing and five washings.

### EXAMPLE 37

A deodorant article was obtained in the same manner as in Example 1 except that the 1 hour of drying at 60°C was changed 40 minutes of drying 110°C and assessed by the acetaldehyde removal test.

The deodorant article was washed once or 5 times in accordance with the washing durability test (JIS L0217 103).

The deodorant article was assessed by the acetaldehyde removal test, as in Example 1, after one washing and five washings.

### EXAMPLE 38

The deodorant article obtained in Example 6 was washed once or 5 times in accordance with the washing durability test (JIS L0217 103).

The deodorant article was assessed by the acetaldehyde removal test, as in Example 1, after one washing and five washings.

### EXAMPLE 39

A deodorant article was obtained in the same manner as in Example 6 except that the 1 hour of drying at 60°C was changed 40 minutes of drying 110°C and assessed by the acetaldehyde removal test.

The deodorant article was washed once or 5 times in accordance with the washing durability test (JIS L0217 103).

The deodorant article was assessed by the acetaldehyde removal test, as in Example 1, after one washing and five washings.

### COMPARATIVE EXAMPLE 3

The deodorant article obtained in Comparative Example 1 was washed once or 5 times in accordance with the washing durability test (JIS L0217 103).

The deodorant article was assessed by the acetaldehyde removal test, as in Example 1, after one washing and five washings.

### COMPARATIVE EXAMPLE 4

A deodorant article was obtained in the same manner as in Comparative Example 1 except that the 1 hour of drying at 60°C was changed 40 minutes of drying 110°C and assessed by the acetaldehyde removal test.

The deodorant article was washed once or 5 times in accordance with the washing durability test (JIS L0217 103).

The deodorant article was assessed by the acetaldehyde removal test, as in Comparative Example 1, after one washing and five washings.

The results of Examples 36 to 39 and Comparative Examples 3 to 4 are shown in Table 3. It is clear from Table 3 that the deodorants of the present invention were more waterproof than conventional deodorants.

**[Table 3]**

| | Deodorant | Drying conditions after deodorant application | Acetaldehyde removal rate [%] | | |
|---|---|---|---|---|---|
| | | | Before washing | After one washing | After 5 washings |
| Ex. 36 | Ex. 1 | 60°C, 1 hour | 99.8 | 99.8 | 99.3 |
| Ex. 37 | Ex. 1 | 110°C, 40 min | 99.9 | 99.2 | 98.7 |
| Ex. 38 | Ex. 6 | 60°C, 1 hour | 99.6 | 94.3 | 86.0 |
| Ex. 39 | Ex. 6 | 110°C, 40 min | 99.7 | 99.7 | 99.5 |
| Com. Ex. 3 | Com. Ex. 1 | 60°C, 1 hour | 40.0 | <5 | <5 |
| Com. Ex. 4 | Com. Ex. 1 | 110°C, 40 min | 41.5 | <5 | <5 |

The entire disclosures of Japanese Patent Application No. 2022-117668 filed on July 25, 2022 and Japanese Patent Application No. 2023-018847 filed on February 10, 2023 including specifications, claims, drawings and summaries are incorporated herein by reference in its entirety.

### INDUSTRIAL APPLICABILITY

The aminooxyalkyl-containing polysiloxane of the present invention can remove aldehydes quickly and persistently, and as a result, can improve the human living environment by decreasing aldehyde-related odors.

## Claims

1. An aminooxyalkyl-containing polysiloxane having a structural unit represented by the following formula (1): 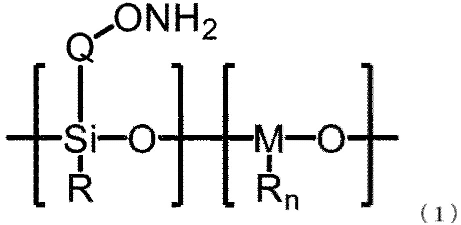 (wherein each R is independently a C₁₋₄ alkyl group, a C₁₋₄ alkoxy group or a hydroxy group, each Q is independently a group selected from the group consisting of -CH₂-, - CH₂CH₂- and -CH₂-Ra-CH₂- (wherein Ra is a C₁₋₁₈ alkylene group in which two or more interspaced -CH₂- groups may each be independently replaced by -O-, -(C=O)O-, - O(C=O)-, -O(C=O)-O-, -C(=O)-NH-, -NH-(C=O)-, -CH=CH- or -C=C-, and in which a hydrogen atom in the alkylene group may be substituted with a C₁₋₃ alkyl group, M is an aluminum atom, a silicon atom or a titanium atom, and n is an integer of 1 or 2, provided that when M is an aluminum atom, n is 1, and when M is a silicon atom or a titanium atom, n is 2).

2. The aminooxyalkyl-containing polysiloxane according to Claim 1, wherein each R is independently a methyl group, a methoxy group, an ethoxy group, a propyloxy group, an isopropyloxy group or a hydroxy group.

3. The aminooxyalkyl-containing polysiloxane according to Claim 1, wherein M is a silicon atom, and n is 2.

4. The aminooxyalkyl-containing polysiloxane according to Claim 1, which has a volume median diameter of at most 300 nm.

5. A method for producing the aminooxyalkyl-containing polysiloxane as defined in Claim 1, which comprises copolymerizing a compound represented by the following formula (2) with a compound represented by the following formula (4) and/or a compound represented by the following formula (5) in the presence of water: 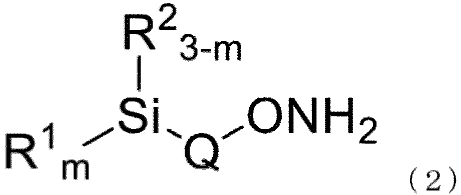 (wherein each R¹ is independently a C₁₋₄ alkyl group, each R² is independently a C₁₋₄ alkoxy group, Q is the same as defined above, and m is an integer of from 0 to 2) 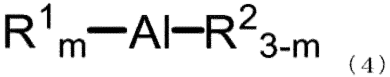 (wherein R¹, R² and m are the same as defined above) 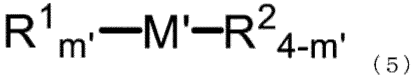 (wherein R¹ and R² are the same as defined above, M' is a silicon atom or a titanium atom, and m' is an integer of from 0 to 3).

6. A method for producing the aminooxyalkyl-containing polysiloxane as defined in Claim 1, which comprises copolymerizing a compound represented by the following formula (2) with a compound represented by the following formula (3) in the presence of water: 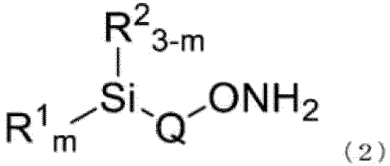 (wherein each R¹ is independently a C₁₋₄ alkyl group, each R² is independently a C₁₋₄ alkoxy group, Q is the same as defined above, and m is an integer of from 0 to 2) 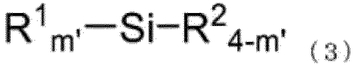 (wherein R¹ and R² are the same as defined above, and m' is an integer of from 0 to 3).

7. The method for producing the aminooxyalkyl-containing polysiloxane according to Claim 5, wherein the ratio (molar ratio) of the total molar amount of the compound represented by the formula (4) and the compound represented by the formula (5) to the molar amount of the compound represented by the formula (2) is such that the total molar amount of the compound represented by the formula (4) and the compound represented by the formula (5) is from 0.001 to 1,000 moles per 1 mole of the compound represented by the formula (2).

8. The method for producing the aminooxyalkyl-containing polysiloxane according to Claim 6, wherein the ratio (molar ratio) of the molar amount of the compound represented by the formula (3) to the molar amount of the compound represented by the formula (2) is such that the molar amount of the compound represented by the formula (3) is from 0.001 to 1,000 moles per 1 mole of the compound represented by the formula (2).

9. A deodorant comprising the aminooxyalkyl-containing polysiloxane as defined in Claim 1.

10. A deodorant article comprising the aminooxyalkyl-containing polysiloxane as defined in Claim 1 and a substrate carrying the aminooxyalkyl-containing polysiloxane.

11. The deodorant article according to Claim 10, wherein the substrate is in the form of fiber, sheet, beads, sponge or board.

12. A method for removing an aldehyde, which comprises bringing the deodorant article as defined in Claim 10 into contact with air containing an aldehyde.
